(19) Europäisches Patentamt
European Patent Office
Office européen des brevets

(11) **EP 2 060 323 A1**

## (12) EUROPEAN PATENT APPLICATION

(43) Date of publication:
**20.05.2009 Bulletin 2009/21**

(51) Int Cl.:
*B01J 23/44* [(2006.01)]     *B01J 23/62* [(2006.01)]
*B01J 37/02* [(2006.01)]     *B01J 37/16* [(2006.01)]
*C07C 5/09* [(2006.01)]      *C07C 7/167* [(2006.01)]
*C22C 1/00* [(2006.01)]

(21) Application number: **07021904.3**

(22) Date of filing: **12.11.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Max-Planck-Gesellschaft zur Förderung der Wissenschaften e.V.
80539 München (DE)**

(72) Inventors:
• **Armbrüster, Marc**
  **Cambridge CB3 9LD (GB)**
• **Schmidt, Marcus**
  **13189 Berlin (DE)**

• **Kovnir, Kirill**
  **01187 Dresden (DE)**
• **Friedrich, Matthias**
  **09496 Marienberg (DE)**
• **Weinhold, Karina**
  **09600 Weissenborn-Berthelsdorf (DE)**
• **Grin, Juri**
  **01277 Dresden (DE)**
• **Schlögl, Robert**
  **10779 Berlin (DE)**

(74) Representative: **HOFFMANN EITLE
Patent- und Rechtsanwälte
Arabellastrasse 4
81925 München (DE)**

(54) **Methods of preparing, optionally supported, ordered intermetallic palladium gallium compounds, the compounds as such, and their use in catalysis**

(57)     The present invention pertains to methods of preparing optionally supported, ordered intermetallic palladium gallium compounds, and the corresponding, optionally supported, intermetallic palladium gallium compounds obtainable by these methods. The present invention also pertains to the use of the optionally supported ordered intermetallic palladium gallium compounds as catalysts, such as in selective hydrogenations of alkynes, in particular ethyne, to give the corresponding alkenes. The optionally supported, ordered intermetallic palladium gallium compounds were found to be highly active and selective catalysts in the above hydrogenation reactions.

**Fig. 1**

**Description**

FIELD OF THE INVENTION

The present invention relates to methods of preparing optionally supported, ordered intermetallic palladium gallium compounds, and the corresponding, optionally supported, intermetallic palladium gallium compounds obtainable by these methods. The present invention also pertains to the use of the optionally supported ordered intermetallic palladium gallium compounds as catalysts, and processes for the selective hydrogenation of an alkyne, in particular ethyne, to give the corresponding alkene using the optionally substituted ordered intermetallic palladium gallium compounds.

BACKGROUND ART

The hydrogenation of acetylene is an important industrial process to remove traces of acetylene in the ethylene feed for the production of polyethylene. Because acetylene poisons the catalyst for the polymerisation of ethylene to polyethylene, the acetylene content in the ethylene feed has to be reduced to the low ppm range. Moreover, economic efficiency requires high selectivity of acetylene hydrogenation in the presence of an excess of ethylene to prevent the hydrogenation of ethylene to ethane.

Typical hydrogenation catalysts contain palladium dispersed on metal oxides. While palladium metal exhibits high activity, *e.g.* in the hydrogenation of acetylene, it possesses only limited selectivity and stability because of the formation of ethane by total hydrogenation and $C_4$ and higher hydrocarbons by oligomerisation reactions. Consequently, improvements in the selectivity to the desired product, such as ethene were highly desired.

The intermetallic compounds PdGa or $Pd_3Ga_7$ are described by E. Hellner et al. in Z. Naturforsch. 2a, 177 - 183 (1947) and by K. Khalaff et al. in J. Less-Common Met. 37, 129 - 140 (1974). Recently, K. Kovnir et al. in Stud. Surf. Sci. Catal., 162, 481 - 488 (2006) uncovered the potential of these materials as highly-selective catalysts for the acetylene partial hydrogenation. In the catalytic tests, the authors used unsupported intermetallic compounds obtained by melting together the necessary amounts of palladium and gallium. Also tested were thus-obtained samples after further treatment in a swing mill and after subjecting them to chemical etching using aqueous ammonia solution. While the activity of the as-made samples was not satisfactory, it could be enhanced by the milling and etching treatment. M. Armbrüster et al., Z. Anorg. Allg. Chem. 632, 2083 (2006) provides for a similar disclosure. Reference can also be made to EP-A-1 834 939 and the corresponding WO 2007/104569. However, the achieved activity still left room for improvements.

In the scientific literature, various approaches have been pursued to prepare ordered intermetallic compounds in finely divided form. For instance, H. Bönnemann et al., in Angew. Chem. Int. Ed. Engl. 29, 273-275 (1990) and in J. Mol. Catal. 86, 129-177 (1994) describe the synthesis of $CoPt_3$, $Co_6Sn_5$ and FeCo by co-reduction of the corresponding metal chlorides in tetrahydrofuran with $LiBEt_3H$. In Langmuir 14, 6654-6657 (1998), H. Bönnemann et al. report the preparation of a tetraoctylammonium-stabilized PtSn colloid with nominal composition $Pt_3Sn$ by co-reduction of the corresponding metal chlorides using $N(Oct)_4[BEt_3H]$ in tetrahydrofuran.

Moreover, there are various reports in the literature on the synthesis of nanoparticulate intermetallic compounds by the reduction of suitable precursors using sodium borohydride ($NaBH_4$) or sodium bis(2-methoxyethoxy)$AlH_2$ as reducing agents. An alternative reducing agent which has been used for the synthesis of nanoparticles is the combination of $NaBH_4$ and tetraethylenglycol, which synthesis method is known as the "modified polyol process".

As reported in Materials Research Bulletin 42, 1969-1975 (2007), K. Page et al. obtained a non-aqueous solution preparation of $Pd_2Sn$ nanoparticles with sizes near 20 nm. They used the metal chlorides, $PdCl_2$ and $SnCl_2$ as starting materials, polyvinylpyrrolidone (PVP) as a stabilizer and tetrabutylammonium borohydride as a reducing agent.

As described in Chem. Mater. 5, 254-256 (1993) J. S. Bradley et al. prepared palladium-copper particles by heating mixtures of palladium acetate and copper acetate hydrate in 2-ethoxyethanol as a reducing alcohol solvent in the presence of polyvinylpyrrolidone as a stabilizer. S. Illy-cherrey et al. in Materials Science and Engineering A283, 11-16 (2000) describe the preparation of finely dispersed nanocrystalline $Pd_xCu_{100-x}$ ($0 \leq x \leq 100$ at.-%) powders by chemical reduction of palladium acetate and copper acetyl acetonate. Sodium hydride in combination with t-BuOH was used as a reducing agent. However, the above papers are silent on any workup of the obtained products. The palladium copper nanoparticles remain dispersed in a solvent or require the presence of a stabilizer to have sufficient storage stability, which renders them unsuitable for use as a catalyst.

However, no reports on the synthesis of nanoparticulate palladium gallium intermetallic compounds have obviously been published yet.

Transport reactions have occasionally been used for the syntheses of ordered intermetallic compounds. It is a characteristic feature of transport reactions for preparing intermetallic compounds that they involve the chemical transport of each of the metals constituting the intermetallic compound.

For instance, H. Schäfer et al. in Z. anorg. allg. Chem. 414, 137-150 (1975) describe the chemical transport of palladium using *e.g.* Cl$_2$, Cl$_2$ + Al$_2$Cl$_6$, Cl$_2$ + Fe$_2$Cl$_6$, and I$_2$ + Al$_2$I$_6$ as transport agents. For instance, experiments in the system Pd/I$_2$, Al$_2$I$_6$ are described as leading to the formation of crystals of Pd$_2$Al. J. Deichsel in his dissertation (University of Hannover, 1998) applied the concept of transport reactions to the palladium gallium system. Using Ga$_2$I$_6$ and Ga$_2$I$_6$ + I$_2$, both, palladium and gallium were transported to give PdGa powder, Pd$_2$Ga and the palladium rich solid solution Pd$_{90}$Ga$_{10}$. Unfortunately, the transport rate in these reactions is low. Like for typical transport reactions it is 100 mg/h at most. Moreover, the method of Deichsel must be carried out in a closed system. The above drawbacks render the method proposed by Deichsel unsuitable for the production of such materials on an industrial scale, where high yields in a short time are desired. Deichsel is also not aware of the catalytic potential of the ordered intermetallic palladium gallium compounds. Furthermore, the dissertation fails to mention the synthesis of supported materials, as well.

M. Binnewies in Angew. Chem. 113, 3801-3803 (2001) converted iron in the form of a spiral wire with silicon tetrachloride to FeSi. The overall reaction is written as: Fe(s) + SiCl$_4$(g) → FeSi(s) + FeCl$_2$(g) Surprisingly, the shape of the starting wire was retained completely. Due to the loss of gaseous FeCl$_2$, FeSi could not be prepared efficiently by the above method in a flow system.

In view of the above prior art, it is an object of the present invention to provide preparation methods for ordered intermetallic palladium gallium compounds, which compounds, optionally in supported form, will have an enhanced activity while maintaining a high selectivity in the selective hydrogenation of alkynes, in particular of ethyne (acetylene) in admixture with a large excess of ethene (ethylene) to afford ethene. The present invention also aims at the provision of methods for the preparation of ordered intermetallic palladium gallium compounds, in particular in supported form, which methods are easy and allow the preparation of these materials on a large scale.

## SUMMARY OF THE INVENTION

The present inventors have surprisingly found that the catalytic activity of ordered intermetallic palladium gallium compounds, for example in the selective hydrogenation of alkynes to give the corresponding alkenes can be increased over the catalysts of the prior art while retaining the excellent selectivity, by preparing these intermetallic compounds by way of specific methods, involving the step of reacting palladium and gallium compounds in the presence of a reducing agent or the step of reacting palladium with a gallium compound in the vapour phase.

Specifically, the present invention pertains to a method of preparing an ordered intermetallic palladium gallium compound (which may be simply referred to as "intermetallic Pd/Ga compound" in this specification) comprising the step of reacting a palladium compound and a gallium compound in the presence of a reducing agent. This embodiment of the present invention is referred to as the "reduction method", hereinafter. The reduction method is suitable to afford nanoparticles of intermetallic Pd/Ga compounds.

According to an alternative embodiment, referred to as the "heterogeneous gas-solid-method", the present invention pertains to a method of preparing intermetallic Pd/Ga compounds comprising the step of reacting palladium, i.e. elemental palladium with a gallium compound, wherein the gallium compound is present in the vapour phase.

Variants of the above methods allow the preparation of supported intermetallic Pd/Ga compounds.

According to another aspect, the present invention relates to the optionally supported intermetallic Pd/Ga compounds as such and their uses as catalysts.

Finally, the present invention pertains to processes for the selective hydrogenation of alkynes to give the corresponding alkenes, in which processes the optionally supported intermetallic Pd/Ga compounds are used as or in a hydrogenation catalyst.

Preferred embodiments of the present invention are subject of the dependent claims.

## BRIEF DESCRIPTION OF THE DRAWINGS

Fig. 1 shows the conversion and selectivity of nano-PdGa (n-PdGa) (2.5 mg) obtained in Example 1, and PdGa (400 mg) obtained in Comparative Example 1, in the hydrogenation of acetylene in admixture with an excess of ethylene at 200 °C.

Fig. 2 shows TEM images of PdGa nanoparticles with a diameter of 5 to 30 nm which have been obtained with the reduction method of the invention.

Fig. 3 shows the conversion and selectivity of nano-Pd$_2$Ga (n-Pd$_2$Ga) (0.1 mg) obtained in Example 2, and Pd$_2$Ga (10 mg) obtained in Comparative Example 2, in the hydrogenation of acetylene in admixture with an excess of ethylene at 200 °C.

Fig. 4a/b shows the conversion and selectivity in the hydrogenation of acetylene in admixture with an excess of ethylene at 200 °C of the following materials: nano-$Pd_2Ga$ (n-$Pd_2Ga$) (0.1 mg) obtained in Example 2, n-$Pd_2Ga$/$SiO_2$ (0.05 mg) obtained in Example 3, and n-$Pd_2Ga$/$Al_2O_3$ (0.05 mg) obtained in Example 4.

Fig. 5 shows the conversion and selectivity of $Pd_2Ga$/C obtained in Example 5 (10 mg), in the hydrogenation of acetylene in admixture with an excess of ethylene at 200 °C.

DETAILED DESCRIPTION OF THE INVENTION

As used herein, the term "ordered intermetallic compound" refers to a compound consisting of two or more metals such as palladium and gallium having an ordered crystal structure. In the ordered crystal structure, substantially all unit cells have the same arrangement of metal atoms.

It will be appreciated that defects which usually cannot be completely avoided in a real crystal may be present in the intermetallic ordered compound. Such defects can cause a small number of unit cells in the ordered intermetallic compound to have an arrangement of metal atoms different from the majority of the unit cells. Defect types include for example vacancies, interstitials, atom substitutions and anti-site defects.

Crystal imperfections due to the presence of defects will lead to a certain homogeneity range of the ordered intermetallic compound. The formulae used in the present specification refer to the ideal crystal structure. As will be appreciated from the above, the stoichiometric ratio of the metals forming the ordered intermetallic compound as indicated in the formula may vary up and down. To give an example, if the ordered intermetallic compound is represented by the general formula $Pd_xGa_y$, then x and y may independently be an integer of 1 or more. In the present specification, PdGa (i.e. x = y = 1) and $Pd_3Ga_7$ represent intermetallic Pd/Ga compounds having a certain stoichiometric ratio of the constituent metals palladium and gallium. Taking account of the above homogeneity ranges the values of x and y may be slightly greater or slightly less than the integers indicated in the formula. The range of the numerical values for the respective ordered intermetallic compound can be taken from the phase diagram of the compound. It corresponds to the respective single-phase region of the intermetallic compound. For instance, it can be taken from the phase diagram of $Pd_3Ga_7$ at 300 °C that the actual value of x in $Pd_xGa_y$ is between 2.99 and 3.06.

The ordered intermetallic compounds as meant in the present invention are to be distinguished from metal alloys and metal solid solutions. Alloys and solid solutions do not have an ordered atomic structure, as described above. Rather, metal atoms are arranged randomly at the atomic positions in the unit cells of alloys and solid solutions.

Ordered intermetallic compounds also generally have a more stable atomic arrangement in comparison to alloys and solid solutions. This results in an enhanced lifetime of catalysts comprising or consisting of ordered intermetallic compounds such as intermetallic Pd/Ga compounds, under reaction conditions. In alloys and solid solutions, atoms are prone to migration with an associated reduction of catalytic performance.

In the reduction method of the present invention, a palladium compound and a gallium compound are reacted in the presence of a reducing agent.

A "reducing agent" as meant in the present application refers to an agent which under the selected reaction conditions, e.g. in the solvent which is optionally present, is capable of reducing the palladium compound and the gallium compound to the zero oxidation state ($Pd^0$ and $Ga^0$). As the standard reduction potential $E^0$ for the reduction of $Ga^{3+}$ or $Ga^+$ to $Ga^0$ is generally more negative than for the reduction of $Pd^{2+}$ to $Pd^0$, it is usually sufficient to make sure that the reducing agent under the selected reaction conditions will convert the gallium compound to $Ga^0$. Then, the palladium compound, which is usually much easier to reduce than the gallium compound, will also be converted to the metallic state (i.e. $Pd^0$ will be formed) thus allowing the formation of the intermetallic Pd/Ga compound.

As will be appreciated, provided the above requirements are met, there are no specific restrictions as to the reducing agent useful in the reduction method of the invention.

However, according to a preferred embodiment, the reducing agent is represented by the following formula (I):

$$M[AR_n(OR')_{3-n}H]_u \qquad (I)$$

wherein M represents an alkali metal or alkaline earth metal atom; A represents Al Ga or B, preferably Ga or B, more preferably B; R and R' are independently selected from $C_{1-6}$ alkyl, $C_{6-10}$ aryl or $C_{7-16}$ aralkyl, preferably from $C_{1-6}$ alkyl, more preferably from $C_{1-3}$ alkyl, i.e. methyl, ethyl or propyl; u is 1 when M is an alkali metal and u is 2 when M is an alkaline earth metal; and n is an integer of 0 to 3. M is preferably an alkali metal and u consequently preferably 1. According to a specific embodiment, n in the above formula is 3 resulting in the following formula (II):

$$M(BR_3H)_u \qquad (II)$$

wherein M, R and u are as defined above, and the preferred meanings of the substituents are also as defined above.

Compounds of the above formulae can be formally regarded as adducts of $MH_u$ and $BR_3$, i.e. $MH_u \cdot (BR_3)_u$. The moiety $BR_3$ in the above formula is preferably $BEt_3$ or $BPr_3$, more preferably $BEt_3$.

Individual examples of reducing agents represented by the above formulae, which can be used with preference include but are not limited to $Na(GaEt_2OEt)H$, $LiBEt_3H$, $NaBEt_3H$, $KBEt_3H$, $Ca(BEt_3H)_2$, $KBPr_3H$, $Na(Et_2BOMe)H$ and $NaB(OMe)_3H$.

The reducing agents represented by the above formulae can be obtained by reacting metal hydrides of the general formula $MH_u$ (u = 1,2) with a complexing agent $BR_3$, $BR_n(OR')_{3-n}$ or $GaR_3$, $GaR_n(OR')_{3-n}$, wherein the substituent meanings are as defined above. Details of the syntheses are given in the paper by P. Binger et al. in Liebigs Ann. Chem. 717, 21-40 (1968) and in DE-A-39 43 351 (and the corresponding EP-A-0 423 627).

When selecting a suitable reducing agent from the group of compounds represented by the above formulae, due consideration is preferably given to the selection of the alkali metal or alkaline earth metal M which should preferably form with the counter ion of the palladium compound and/or gallium compound a product which will readily dissolve in the solvent in which the reduction may be carried out.

The most preferred reducing agent is $LiBEt_3H$. This is because it is commercially available as a solution in tetrahydrofuran (THF) under the tradename Superhydride® (Aldrich, 1.0 M lithium triethylborohydride in THF). Superhydride® is also advantageous in that no solid residues will remain after the reaction, which facilitates the workup of the reaction mixture.

Further suitable reducing agents for use in the reduction method of the present invention may for instance be lithium aluminum hydride ($LiAlH_4$) and derivatives in which one to three hydrogen atoms are replaced with organic substituents such as $C_{1-6}$ alkyl or $C_{6-10}$ aryl.

Tetraalkylammonium borohydrides of the general formula $R_4NBH_4$, in which R is the same or different and independently selected from alkyl, such as $C_{1-20}$, preferably $C_{1-14}$, more preferably $C_{1-10}$ alkyl, may also be used as reducing agents. An example is $(C_4H_9)_4NBH_4$. Like in the case of lithium aluminium hydride, one to three hydrogen atoms in the tetrahydroborate moiety $BH_4^-$ included in $R_4NBH_4$ may be substituted with an organic residue such as $C_{1-6}$ alkyl, preferably $C_{1-3}$ alkyl, or $C_{6-10}$ aryl. An example of such a tetrahydroborate moiety is $BEt_3H^-$. Concrete examples of tetraalkylammonium borohydrides which contain that moiety and may be used in the reduction method of the invention are $N(octyl)_3MeBEt_3H$, $NBu_4BEt_3H$, $N(hexyl)_4BEt_3H$, $N(octyl)_4BEt_3H$, and $N(decyl)_4BEt_3H$.

Still further reducing agents for use in the reduction method of the invention may be alkali metals, such as lithium, sodium or potassium, as well as naphthalides, such as sodium naphthalide.

The palladium compound for use in the reduction method of the present invention is not particularly limited in kind, as long as it can be reduced to the metallic state (*i.e.* $Pd^0$) by the reducing agent. Both, Pd (II) and Pd (IV) compounds are useful, with Pd (II) compounds being preferred. To avoid any unnecessary consumption of the reducing agent, the counter ion of the palladium compound will preferably not be reduced by the reducing agent under the selected reaction conditions. Suitable compounds are Pd (II) halides, such as $PdCl_2$, $PdBr_2$, $PdI_2$ and $PdF_2$, palladium cyanide, palladium sulfate, palladium nitrate, palladium acetate, palladium trifluoroacetate, palladium propionate and palladium acetylace-tonate ($Pd(acac)_2$), with the latter being preferred.

The gallium compound for use in the reduction method of the present invention is also not particularly limited in kind, as long as it is capable of being converted to $Ga^0$ by the reducing agent, and is capable of forming, along with the palladium compound and in the presence of the selected reducing agent, an ordered intermetallic palladium gallium compound. The gallium compound may be a gallium (I) or gallium (III) compound. Preferably it is a gallium (III) compound. Like in the case of the palladium compound, the counter anion in the gallium compound will preferably not be reduced or otherwise react with the reducing agent used. For this reason, gallium trihalogenides, in particular $GaCl_3$, $GaBr_3$ and $GaI_3$ and also gallium sulfate can be exemplified as gallium compounds for use in the reduction method of the present invention. While hydrates such as $Ga(NO_3)_3 \cdot 8H_2O$ can be used as gallium compounds, they are less preferred since a larger amount of reducing agent may be necessary due to the reaction with hydrate water and the concomitant formation of hydroxides which have to be removed, e.g. by washing. Preferably, the gallium compound is a gallium halide such as $GaCl_3$, $GaBr_3$ or $GaI_3$, most preferably it is $GaCl_3$.

The reduction method of the present invention is preferably carried out in a solvent. In particular, when Super-rhydride® is used as the reducing agent, the solvent is preferably tetrahydrofuran. This is because $LiBEt_3H$ is sold in a THF solution under the trademark Superhydride®. However, tetrahydrofuran is also a suitable solvent for other reducing agents for use in the present invention, e.g. those of formula (I) or tetraalkylammonium borohydrides. In more general terms, useful solvents in the reduction method of the invention are aprotic solvents. Apart from THF, diglyme, *i.e.* diethylenglycol dimethyl ether can be exemplified, and may be used *e.g.* when $Ca(BEt_3H)_2$ is used as a reducing agent.

When $LiAlH_4$ or derivatives thereof are used as a reducing agent, tetrahydrofuran, dioctylether, octadecan and propylene carbonate can be used as solvents, preferably after drying.

Most preferably, the reduction method of the present invention is carried out in boiling tetrahydrofuran (after drying) using $Pd(acac)_2$ and $GaCl_3$ as palladium and gallium starting compounds, respectively, and Superhydride® as a reducing agent.

To avoid any reaction of the reducing agent with moisture or oxygen, the reaction, more specifically step (1) of the reaction method is preferably carried out under a protective atmosphere, *e.g.* in nitrogen or argon, and in solvents (if any) which were previously dried.

In step (1) of the reduction method, the reducing agent may be added to the palladium compound and the gallium compound being simultaneously present, e.g. to a solution of both, the gallium and the palladium compound. In the alternative, the reducing agent may be added to the gallium compound, *e.g.* the gallium halide (in particular $GaCl_3$) prior to addition to the palladium compound, e.g. dissolved in a solvent. When step (1) is carried out in a solvent, the palladium compound (*e.g.* $Pd(acac)_2$) may be dissolved in the solvent, *e.g.* THF, prior to adding the solution (*e.g.* THF solution) of the gallium compound and the reducing agent. As the present inventors found, the above procedure allows for an adjustment of the particle size (such as the nanoparticle size) of the resultant intermetallic Pd/Ga compound by heating the solution of the palladium compound (such as $Pd(acac)_2$) in the solvent, *e.g.* THF, at a suitable temperature, prior to adding the mixture of gallium compound and reducing agent.

The intermetallic Pd/Ga compound prepared in the reaction method of the present invention is preferably selected from the group consisting of $PdGa$, $Pd_2Ga$, $PdGa_5$, $Pd_3Ga_7$ and $Pd_5Ga_3$, or a mixture thereof. More preferably, it is $PdGa$, $Pd_2Ga$ or $Pd_3Ga_7$, and most preferably $PdGa$.

In the reduction method of the present invention, a specific intermetallic Pd/Ga compound can be obtained by suitable selection of the molar ratio of palladium and gallium present in the palladium and gallium compound. Using $Pd(acac)_2$ as the palladium compound, $GaCl_3$ as the gallium compound, $LiBEt_3H$, *i.e.* Superhydride®, as the reducing agent, and THF as a solvent the approximate molar ratios summarized in the following table were found to lead to specific intermetallic compounds.

| Intermetallic Pd/Ga compound | Equivalents $Pd(acac)_2$ | Equivalents $GaCl_3$ |
|---|---|---|
| $PdGa$ | 1 | 2 |
| $Pd_2Ga$ | 1 | 1.25 |
| $Pd_3Ga_7$ | 1 | 3 |

As can be seen from the above table, the gallium present in the $GaCl_3$ starting compounds were incorporated in the intermetallic Pd/Ga compound, incompletely. Without wishing to be bound by theory, this may be explained by the formation of an adduct of the solvent THF and $GaCl_3$. It was also found that 9 equivalents $LiBEt_3H$ were needed for one equivalent of $GaCl_3$. Possibly, this is due to a partial reduction of the solvent THF by $LiBEt_3H$.

In the reaction method of the invention, the ordered intermetallic palladium gallium compound can be isolated in powder form subsequent to the reaction in step (1). If step (1) is carried out in a solvent, a suspension is typically obtained, and the intermetallic Pd/Ga compound can be separated from the suspension by way of methods which are usual for separating solids from suspensions. Centrifugation, decanting and drying (optionally under vacuum) may be mentioned here. Of course, the separating steps for isolating the intermetallic Pd/Ga compound in powder form can be used in combination. Moreover, washing steps can be carried out in between and/or after the separating steps. For example, the suspension obtained in step (1) can be subjected to centrifugation and subsequent decanting, washing of the thus-obtained solids, *e.g.* in tetrahydrofuran, and subsequent drying, for instance in vacuum.

The reduction method in accordance with the invention allows the preparation of nanoparticles of the ordered intermetallic palladium gallium compound. As meant herein, nanoparticles have an average diameter in the nanometer range, *i.e.* from 1 nm to below 1000 nm. Preferably, the nanoparticles have an average diameter of 1 to 100 nm.

The intermetallic Pd/Ga compound in powder form obtainable by the reduction method of the invention may have a content of nanoparticles of $\geq 50$ wt.-% in terms of the overall weight of the powdered intermetallic compound. Preferably, the content is $\geq 80$ wt.-%, more preferably $\geq 90$ wt.-%, most preferably $\geq 95$ wt.-% and especially $\geq 99$ wt.-%, in all cases in terms of the overall weight of the intermetallic Pd/Ga compound in powder form.

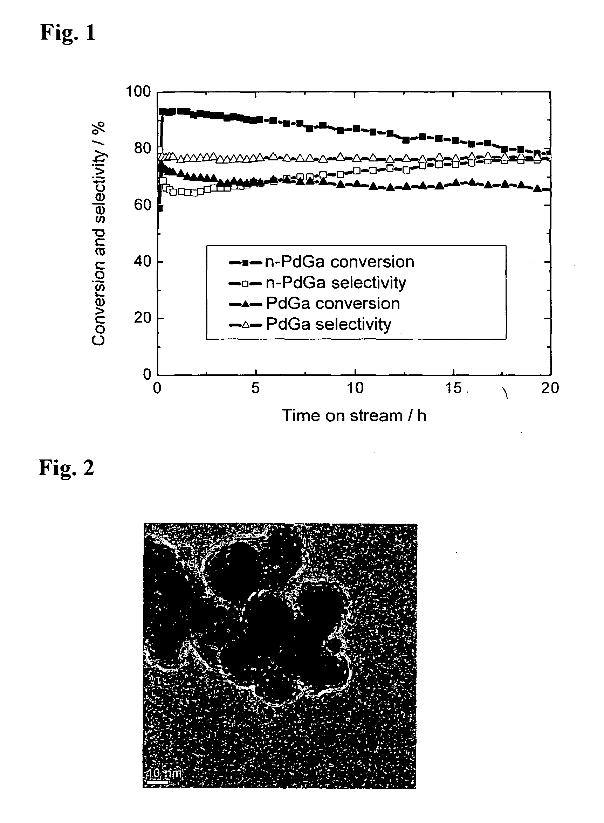

The nanoparticulate form of the obtained intermetallic Pd/Ga compounds can be confirmed by X-ray powder diffractometry as well as transmission electron microscopy. By determining the FWHM of the reflections in the XRD pattern (prepared under argon atmosphere) and applying the Scherrer equation, the crystallite size of the intermetallic Pd/Ga compounds prepared by the reduction method of the invention can be calculated. The results are in full conformity with those obtained from TEM (transmission electron microscopy) pictures. A typical TEM picture of a PdGa sample obtained after tempering at 185 °C is shown in Fig. 2. The diameters of the PdGa nanoparticles are within a range of 5 to 30 nm.

The reduction method of the present invention allows the preparation of intermetallic Pd/Ga compounds in nanoparticulate powder form without the need of any stabilizer. While surface active substances which are generally used in the art for stabilizing nanoparticles, such as alcohols, diols, polymers such as PVP and PVA and glycols such as ethylene glycol, as well as citrates and AOT (sulfobutanedioic acid bis(2-ethylhexyl ester) sodium salt) may be used in the present invention, they are not necessary. In view of the catalytic application of the intermetallic Pd/Ga compounds, this is highly advantageous since the stabilizers do not have to be removed. While stabilizers such as PVP can be removed by appropriate heat treatment, this involves the risk of sintering. For these reasons, the reduction method in accordance with the present invention which is capable of affording nanoparticles is preferably carried out in the absence of any stabilizer.

The ordered intermetallic palladium gallium compounds obtained in step (1) of the reduction method of the present invention may be obtained as a phase mixture of more than one kind of ordered intermetallic palladium gallium compounds, in accordance with the phase diagram, provided the system is in thermodynamic equilibrium. With the purpose of obtaining the desired intermetallic Pd/Ga compound as a single phase material, the material obtained in step (1) is preferably subjected to a tempering step (3). The tempering is preferably carried out after isolating the intermetallic Pd/Ga compound in accordance with step (2), e.g. in powdered, in particular nanoparticulate form.

The tempering temperature is not particularly limited, provided it is below the decomposition temperature of the intermetallic Pd/Ga compound. Upon tempering, the size of the particles of the intermetallic Pd/Ga compound (powder) will usually increase with increasing tempering temperature. In view of the above, the tempering temperature may be in a range of 160 to 200 °C. It is preferably in a range of 180 to 190 °C, preferably about 185 °C.

To avoid any agglomeration events from occurring, in particular when the intermetallic Pd/Ga compound is present in nanoparticulate form, the tempering is preferably carried out in a solvent. When a solvent is used, the upper limit of the tempering temperature may be limited by the boiling point of the solvent. At the same time, the melting point of the solvent may define the lower limit of the tempering temperature. Also, the solvent should preferably be inert under the tempering conditions. In this context "inert" is intended to indicate that the solvent does not react with, or deteriorate, the ordered intermetallic Pd/Ga compound to be tempered, while at the same time the solvent is not decomposed due to the catalytic activity of the intermetallic Pd/Ga compound. Taking account of the above, the solvent may be selected from hydrocarbons, glycols, ethers, and mesitylene, each having a boiling point above the selected tempering temperature. In view of the above preferred tempering temperatures the solvent is preferably a $C_{10-16}$, more preferably a $C_{10-14}$ linear, branched or cyclic hydrocarbon. According to another preferred embodiment, the tempering solvent is a di($C_{4-10}$) ether, more preferably a di($C_{6-9}$)ether, most preferably dioctylether.

There are no restrictions as to the tempering time and it may for instance be in the range of 0.5 to 6 h, preferably, it is 2 to 5 h and more preferably 3 to 4 h.

Intermetallic Pd/Ga compounds can also be prepared by the reduction method of the invention in supported form. Specifically, a support material is impregnated with a solution or suspension of a palladium compound and a gallium compound, and the obtained impregnated support is subjected to a reaction in the presence of a reducing agent as defined above. The palladium compound and the gallium compound may be used in the form of separate solutions and/or suspensions, or as a solution or suspension comprising both, the palladium compound and the gallium compound. Dependent on the used palladium and/or gallium compounds, suitable liquids for dissolving or suspending these compounds can be employed. The impregnation of the support may be carried out in accordance with usual methods in the field of catalysis, such as incipient wetness impregnation. The support material to be impregnated is preferably inert to the reactants, in particular the reducing agent, and at the same time preferably provides a sufficiently high surface area, such as $\geq 50$, preferably in the range of 50 to 1000, more preferably in the range of 50 to 500 $m^2/g$ (determined in accordance with the BET method using nitrogen as an adsorbent). To avoid that any reducing agent, such as Superhydride® is consumed due to the presence of moisture, the support material is preferably carefully dried prior to impregnation. Without limitation, silica, alumina and carbon can be exemplified as support materials.

As will be appreciated, all the above embodiments of the reduction method of the invention can also be employed for the preparation of the supported counterparts of the intermetallic Pd/Ga compounds, if appropriate.

The optionally supported intermetallic Pd/Ga compounds of the present invention are highly useful as catalysts. For instance, they may be used as catalysts in the, preferably selective, hydrogenation of at least one unsaturated hydrocarbon compound as detailed in WO 2007/104569.

The skilled person in the field of hydrogenation catalysis will readily select and optimise the reaction parameters for a certain selective hydrogenation reaction. For instance, the temperature range of industrial selective hydrogenations is typically 10 ° to 300°C, preferably 20 ° to 250 °C, most preferably 30 ° to 200 °C. The pressure is generally 1 to 100 bar, preferably 2 to 75 bar, most preferably 5 to 50 bar. For more details, reference is made to WO 03/106020.

The (optionally supported) intermetallic Pd/Ga compounds of the invention proved to be particularly active and selective catalysts in a process for the selective hydrogenation of an alkyne to give the corresponding alkene, also referred to as the semihydrogenation of the alkyne. A hydrogenation catalyst comprising an intermetallic Pd/Ga compound in accordance with the present invention, e.g. in a proportion of $\geq 20$ wt.-%, preferably $\geq 50$ wt.-%, more preferably $\geq 80$ wt.-%, still more preferably $\geq 90$ wt.-% and most preferably $\geq 95$ wt.-% was found to be highly selective to the desired alkene, in particular in the hydrogenation of acetylene (ethyne) to ethene, even when the ethyne is present in admixture with a large excess of ethene in the reaction mixture.

Generally, a hydrogenation of an alkyne is referred to as being selective if the triple bond is hydrogenated with preference only once, and the further reaction to the single bond is hardly observed, i.e. if the semihydrogenation of the triple bond is predominant. For the purpose of the present invention, the hydrogenation of an alkyne is referred to as selective if the molar ratio of the desired target compound, *e.g.* the corresponding alkene, to the undesired target compound, e.g. the corresponding alkane, is larger than 1 : 1, preferably more than 2 : 1, more preferably more than 5 : 1, and most preferably more than 10 : 1.

The alkyne to be converted in the selective hydrogenation process of the invention may be an alkyne, dialkyne, trialkyne or polyalkyne. Preferably, it is an alkyne, *i.e.* a hydrocarbon compound containing only a single triple bond.

The alkyne is preferable ethyne (acetylene), and this is the most preferred embodiment of the present invention. Through the process for the selective hydrogenation of the invention, ethyne will predominantly be converted to ethene (ethylene) while the hydrogenation of ethene to afford ethane is negligible. This is even so when the selective hydrogenation of ethyne is carried out under reaction conditions where ethyne is present in admixture with an excess of ethene in relation to ethyne, which is a particularly preferred embodiment of the selective ethyne hydrogenation according to the present invention. Most preferably, ethene is present in the reaction mixture to be hydrogenated in a large excess in relation to ethyne. The ethyne to ethene weight ratio in the starting mixture of the selective ethyne hydrogenation of the invention is preferably 1 : 10 to 1 : $10^6$, more preferably 1 : 50 to 1 : $10^3$. In industrial processes, the ethene to ethyne weight ratio in the mixture obtained after the selective hydrogenation is typically as large as > $10^6$.

The selective hydrogenation of phenyl acetylene to styrene in excess of styrene is another example of a selective hydrogenation of an alkyne. As will be appreciated, that reaction is the polystyrene counterpart of the selective acetylene hydrogenation in excess of ethylene in the feed used for the preparation of polyethylene.

As shown in the working examples and illustrated in the figures, the intermetallic compounds of the present invention, in a hydrogenation reaction of acetylene simulating the industrial hydrogenation of acetylene exhibit an improved activity while retaining the high selectivity to ethene without further hydrogenation to ethane, in comparison to conventional intermetallic Pd/Ga compounds obtained by melting together the constituent metals.

It is surprising that the excellent selectivity of the catalyst is retained and the activity further increased by using nanoparticulate intermetallic Pd/Ga compounds in place of the conventional materials such as *e.g.* described in WO 2007/104569. The selectivity of a catalyst is generally directly connected to the number of different active sites present. In nanoparticles, the number of edges, kinks and corners is increased over the bulk material. Consequently, the selectivity of nanoparticulate material can be expected to be lower due to the larger variety of sites in comparison to bulk material comprising larger particles. To the surprise of the present inventors, the usage of nanoparticulate intermetallic Pd/Ga compounds substantially retained the selectivity of the conventional material of a larger particle size.

It was unexpectedly found that both, the long-term activity (as evidenced by the conversion) and the selectivity in the selective hydrogenation of acetylene in an excess of ethene can be further increased for intermetallic Pd/Ga compounds in powder form, in particular in nanoparticulate form, by suspending this in an inert liquid in the presence of an inert material, and subsequent removal of the liquid.

In this context, a liquid and a material is/are referred to as "inert", if they do not react with or otherwise alter, e.g. deteriorate the properties of the intermetallic compound. The removal of the inert liquid can be achieved by usual methods. Examples of such methods are without limitation heating at above the boiling point of the liquid (optionally in vacuum). To prevent any sintering or agglomeration events of the intermetallic Pd/Ga compound in powder form, in particular in nanoparticulate form, upon removing the liquid, the boiling point of the inert liquid should preferably be low such as ≤ 100 °C, preferably ≤ 90 °C. The inert liquid may for instance be a hydrocarbon, in particular a $C_{5-10}$, preferably a $C_{6-8}$ linear, branched or cyclic hydrocarbon, such as hexane or cyclohexane. Moreover, the inert liquid may be THF, alcohols, diols, ethyleneglycol, toluene and benzene. Of course, mixtures of inert solvents can also be used.

As used herein, the inert material is intended to mean any material that is substantially, preferably completely devoid of any catalytic activity in the reaction, *e.g.* the selective alkyne hydrogenation to be catalyzed. Consequently, the inert material, when subjected to a blank catalytic measurement preferably does not show any (substantial) catalytical activity in the reaction at issue. In the present invention, the inert material is preferably used in the form of powder.

Examples of the inert material for use in the present invention are silica, silica gel, kieselguhr and silicates. Moreover, alumina, titania, zirconia, zeolites, active carbon, talc, kaolin, boron nitride, barium carbonate, barium sulphate, calcium carbonate, strontium carbonate, aluminium nitride and clays can be exemplified.

Preferably, the inert material has a high specific surface area that is typically in the range of ≥ 10, preferably 10 to 2000 $m^2$/g, e.g. 100 to 1500 $m^2$/g, and especially 300 to 1200 $m^2$/g.

According to a preferred embodiment, the inert material is silica, in particular silica having a specific surface area of 100 to 500 $m^2$/g. Suitable types of silica having a high surface area are commercially available. Examples are γ-SiO$_2$ (Degussa AG) and various types of Aerosil®. For the purpose of the present specification, the specific surface area of the materials refers to the specific surface area as measured according to the BET method using nitrogen as an adsorbent.

According to another preferred embodiment, the inert material is alumina. While the alumina is not specifically limited in kind, it is preferably a type of alumina having a specific surface area of 100 to 500 $m^2/g$. As regards the pH, neutral, acidic or basic alumina may be used, with neutral alumina being preferred. Suitable means such as agitation or ultrasonic treatment may be used to assist the suspension of the intermetallic compound in the inert liquid.

It was found out by the present inventors that the reduction method as meant in the present application is also useful for the preparation of a further class of ordered intermetallic compounds exerting high activity and activity as catalysts in hydrogenation reactions such as the selective (semi)hydrogenation of acetylene to afford ethylene in the presence of a large excess of ethylene. This further class of ordered intermetallic compounds are Pd/Cu compounds. This is another aspect of the present invention.

All of the above general statements on the reduction method to prepare intermetallic Pd/Ga compounds are likewise applicable to the intermetallic Pd/Cu compounds, except of course that a copper compound is used in place of the gallium compound. In view of the above, the present invention according to another aspect is concerned with a method of preparing an ordered intermetallic palladium copper compound comprising the step (1) of reacting a palladium compound and a copper compound in the presence of a reducing agent.

Useful palladium compounds and reducing agents are as explained above for the reduction method of preparing the intermetallic Pd/Ga compounds. The copper compound may be a copper (II) or a copper (I) compound, with copper (II) compounds being preferred. Useful copper compounds are copper(II)acetate, $Cu(acac)_2$, copper halides such as $CuCl_2$, $CuBr_2$ or $CuI_2$, copper sulfate, and copper(II) trifluoracetylacetonate. $Cu(acac)_2$ is a preferred copper compound, and this is preferably used together with $Pd(acac)_2$ as a palladium compound.

Using $LiBEt_3H$ in the form of Superhydride® as a reducing agent, the intermetallic Pd/Cu compounds $Cu_{85}Pd_{15}$ and $Cu_{60}Pd_{40}$ can for instance be obtained as described in the reference examples. The temperature of step (1) of the reduction method for this aspect of the invention turned out to be preferably about 50 °C.

As will be appreciated from the above, the reduction method of the present invention using Superhydride® as a reducing agent allows for the preparation of nanoparticles of intermetallic Pd/Cu compounds, *e.g.* nanoparticulate $Cu_{g5}Pd_{15}$ or nanoparticulate $Cu_{60}Pd_{40}$ in the form of dry powders without need of any stabilizer. As such, the above nanoparticulate powders can be used as catalysts, such as in the selective hydrogenation of acetylene. This is different from the scientific literature discussed in the background art section of this specification.

Another method of preparing an intermetallic Pd/Ga compound in accordance with the present invention is a method which comprises the step of reacting palladium with a gallium compound, the gallium compound being in the vapour phase. This method is referred to as the "heterogeneous gas-solid-method". The gallium compound in the vapour phase is occasionally referred to as the "gaseous gallium compound", hereinafter.

The palladium to be reacted is intended to mean elemental palladium. It can be used in various forms such as palladium wire, palladium mesh, palladium powder and supported palladium. The use of supported palladium is particularly advantageous in that it allows the manufacture of supported intermetallic Pd/Ga compounds using commercially available supported Pd materials as raw materials. Of course, these raw materials can also be made, *e.g.* by incipient wetness of support materials. The support should preferably be inert to the extent that it is devoid of any reactive groups such as OH groups capable of reacting with the gallium compound in the vapour phase. For instance, it may be burnt alumina without substantial amounts of OH groups on the surface. Alternative inert supports are carbon, aluminium nitride, zirconia. Preferably, it is carbon, such as activated carbon. Mixtures of materials can also be used.

The amount of intermetallic Pd/Ga compounds in the supported ordered intermetallic palladium compounds of the invention may for instance be in the range of 0.5 to 10 wt.% and it is preferably in the range of 1 to 5 wt.%, based on the total weight of the material, e.g. intermetallic compound and support. The above exemplary ranges by weight are also applicable to the corresponding supported intermetallic Pd/Ga compounds prepared by way of the reduction method involving the impregnation of a (preferably inert) support material with a solution or suspension of a palladium compound and a gallium compound.

In the heterogeneous gas-solid-method of the invention, the reaction of the palladium with the gallium compound in the vapour phase is triggered by the formation of the ordered intermetallic palladium gallium compound. The gallium compound being in the vapour phase may be fed to the palladium, which remains immobile, where it reacts with the palladium to give the intermetallic Pd/Ga compound.

The gaseous gallium compound is not particularly limited in kind and may be a gallium halogenide, in particular a gallium iodide such as $GaI$ or $GaI_3$, a gallium bromide such as $GaBr$ and $GaBr_3$, and a gallium chloride, such as $GaCl$ and $GaCl_3$. $GaH_3$ may also be used. Preferably, the gallium compound in the vapour phase is a gallium halogenide, in particular a gallium iodide, such as $GaI$ or $GaI_3$, or a mixture thereof.

For instance, $Pd_2Ga$ can be formed using any of the above-exemplified gallium iodides, gallium bromides or gallium chlorides as the gallium compound in the vapour phase.

The gaseous gallium compound may be formed in a first reaction zone at a temperature $T_1$, and the reaction of palladium with the said compound may take place in a second reaction zone at a temperature $T_2$, which is higher than $T_1$. While the heterogeneous gas-solid-method may be carried out in a closed system, it is preferably carried out in an open flow system. Carrying out the method in an open system, in particular a flow system is preferred in that it allows the direct formation of intermetallic Pd/Ga compounds supported on an inert support material as mentioned above. This is different from corresponding methods of the prior art, which cannot be carried out in an open system.

The material from which the gallium compound in the vapour phase is formed in the first reaction zone is not particularly limited in kind. In the embodiment where a gallium iodide is the gallium compound in the vapour phase, the material may comprise a gallium and an iodine source. The gallium source may be $GaEt_3$, and the iodine source may be $HgI_2$, $PdI_2$ or $MeI$. Preferably, a stream of $I_2$ vapour flowing over elemental gallium forms the gallium iodide in the vapour phase, e.g. $GaI$ or $GaI_3$. According to another preferred embodiment, mixtures of Ga and $GaI_3$ are used as a raw material for forming the gaseous gallium iodide.

The temperature $T_1$ is selected such that the gallium compound is present in the vapour phase. Consequently, $T_1$ may for instance be in the range of 200 to 1300 °C, more preferably 500 to 650 °C; most preferably it is about 600 °C (873 K). The temperature $T_2$ is preferably selected such that the vapour pressure of palladium is negligible, and that palladium does not form any volatile compounds, such as iodides at that temperature. The maximal temperature of $T_2$ (and also $T_1$) should preferably be below the temperature at which the intermetallic Pd/Ga compound starts to melt, deteriorate and/or decompose. As the melting point of PdGa is about 1065 °C, $T_2$ is preferably below that temperature when this compound is to be formed. In the case of $Pd_2Ga$, the corresponding temperature (melting point) is about 1265 °C. In view of the above, $T_2$ may be in the range of 400 to 1300 °C and preferably 700 to 850 °C; and it is most preferably about 800 °C (1073 K). The difference between $T_2$ and $T_1$ may be in the range of 100 to 300 K, preferably it is 150 to 250 K and most preferably about 200 K.

The particular ordered intermetallic palladium gallium compound formed in the heterogeneous gas-solid-method can be determined in the case of gallium iodide being the gaseous gallium compound by the molar gallium : iodine ratio. For instance, the Ga : I molar ratio may be varied in the range of 1 : 3 to 1.2 : 1 for the formation of $Pd_2Ga$. PdGa may be formed at a Ga : I molar ratio of 1.4 : 1 to 1.5 : 1.

The intermetallic Pd/Ga compound obtained in the heterogeneous gas-solid-method may be subjected to a subsequent etching treatment. The etching of the surface of the ordered intermetallic palladium gallium compound may be achieved by chemical etching, *e.g.* using complexing amines, such as EDTA and derivatives, preferably by using alkaline etching solutions. Useful alkaline etching solutions are for example aqueous alkali hydroxide (*e.g.* sodium and potassium hydroxide), alkaline earth hydroxide solutions and aqueous ammonia solutions. Alkali etching solutions having a pH in the range of 8.0 to 14, such as 9.0 to 13.5, preferably 9 to 10, in particular those prepared using aqueous ammonia solutions are preferred.

It was surprisingly found that intermetallic Pd/Ga compounds prepared by way of the heterogeneous gas-solid-method of the invention can be further improved in terms of selectivity in the semihydrogenation of acetylene in comparison to their non-etched counterparts by the etching treatment, preferably using an alkaline solution.

According to particularly preferred embodiments of the heterogeneous gas-solid-method of the invention, gaseous gallium iodide (GaI and/or $GaI_3$) is formed in an open system either from a mixture of Ga and $GaI_3$, or by contacting $I_2$ vapour with elemental gallium, and the gaseous gallium iodide is reacted with supported palladium (in particular Pd/C) to give a supported intermetallic Pd/Ga compound, in particular carbon-supported PdGa or $Pd_2Ga$, which is preferably subsequently etched at a pH of e.g. 9 or 10 (for instance using aqueous ammonia solution).

The following examples are given for illustration of the present invention and must not be construed in a limiting sense.

EXAMPLES

Prior to usage, the THF (99.9 %, Roth) was dried and distilled over $CaH_2$ (95 %, Fluka) under protective argon atmosphere. The other chemicals were used without further treatment. All procedures were carried out under protective argon atmosphere in a glove box with oxygen and water levels below 0.1 ppm.

PREPARATION EXAMPLES

EXAMPLE 1 Preparation of nano-PdGa (n-PdGa) with the reduction method

For the synthesis of PdGa, 0.1003 g (1 eq.) of $Pd(acac)_2$ (purum, Fluka) was given in a 100 ml 3-neck reaction flask. In a separate vessel, 0.1163 g (2.0061 eq.) of $GaCl_3$ (99.999 %, AlfaAesar) were added to 3.28 ml (20 eq.) Superhydride® (1.0 M in THF, Aldrich), which addition was accompanied by an exothermic reaction resulting in a clear, colourless solution. This solution (solution (2)) was filled into a syringe, placed in a perfusor and connected to the 3-neck flask by a hypothermic needle through a septum. 10 ml of THF were added to the $Pd(acac)_2$ in the 3-neck flask, together with a magnetic stir bar resulting in a clear yellow solution (solution (1)). After this, a water-cooler was installed on top of the flask, while the third neck held a thermocouple in gas-tight connection. During the reaction, a low stream of argon was passed through the top of the cooler to allow for the disposal of gaseous reaction products.

After starting the magnetic stirrer, solution (1) was heated to reflux (66 °C) within 6 min. Then the solution (2) was added by the perfusor with a speed of 149.1 ml/h resulting in an immediate change of the yellow solution to a black suspension. After 2.6 min the addition was complete. The resulting suspension was refluxed for 4 h in total before heating, cooling and argon are switched off.

The suspension was centrifuged at 6000 rpm for 10 min, resulting in a black precipitate and an amber, clear solution which was decanted and discarded. The precipitate was washed three times with 2 ml of THF with centrifugation and decanting in between. Subsequently the precipitate was dried at a pressure of 100 mbar until constant weight was reached (20 min) which was 0.0625 g, corresponding to a yield of 77.1 %.

After adding 15 ml of dioctylether (99 %, Aldrich) and a magnetic stir bar to the intermediate product in a 100 ml 2-neck flask and after installing cooler and thermocouple, the resulting suspension was stirred and heated to 185 °C in 13 min. This temperature is then held for 4 h. After cooling down to ambient temperature, the suspension was processed like before, yielding 0.05625 g, corresponding to a yield of 69.4 %.

Fig. 1 shows the results of the catalytic testing of 2.5 mg of the obtained nano-PdGa in the selective hydrogenation of acetylene as detailed hereinafter.

EXAMPLE 2: Preparation of nano-$Pd_2Ga$ (n-$Pd_2Ga$) with the reduction method

For the preparation of nanoparticulate $Pd_2Ga$, the method of Example 1 was repeated except for adjusting the amount of $Pd(acac)_2$, $GaCl_3$ and superhydride®. Specifically, to obtain $Pd_2Ga$, 1.25 eq. $GaCl_3$ and 1 eq. $Pd(acac)_2$ have to be used.

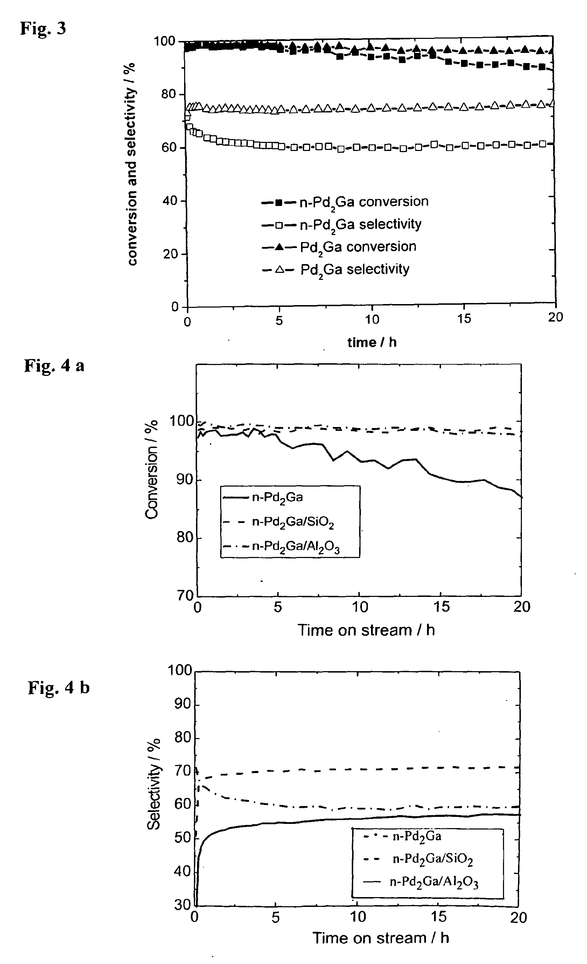

Fig. 3 shows the results of the catalytic testing of 0.1 mg of the obtained nano-$Pd_2Ga$ in the selective hydrogenation of acetylene as detailed hereinafter.

EXAMPLE 3: Preparation of nano-$Pd_2Ga/SiO_2$ (n-$Pd_2Ga/SiO_2$)

5 mg nano-$Pd_2Ga$ as obtained in Example 2 was suspended in 20 ml of cyclohexane via ultrasonic treatment (15 minutes). 71.25 mg of dry silica was added to the suspension and subjected to ultrasonic treatment for additional 15 minutes. The resulting mixture was dried in the hood until most of cyclohexane was evapourated (2 days). Finally, the resulting dark powder was further dried in a desiccator under vacuum (3 mbar) for 2 hours.

Figs. 4a and 4b show the results of the catalytic testing of 0.05 mg of the obtained nano-$Pd_2Ga/SiO_2$ in the selective hydrogenation of acetylene as detailed hereinafter.

EXAMPLE 4: Preparation of nano-$Pd_2Ga/Al_2O_3$ (n-$Pd_2Ga/Al_2O_3$)

Example 3 was repeated except that alumina powder (neutral alumina, Aldrich) was used in place of silica.

Figs. 4a and 4b show the results of the catalytic testing of 0.05 mg of the obtained nano-$Pd_2Ga/Al_2O_3$ in the selective hydrogenation of acetylene as detailed hereinafter.

EXAMPLE 5: Synthesis of $Pd_2Ga/C$ with the heterogeneous gas-solid-method

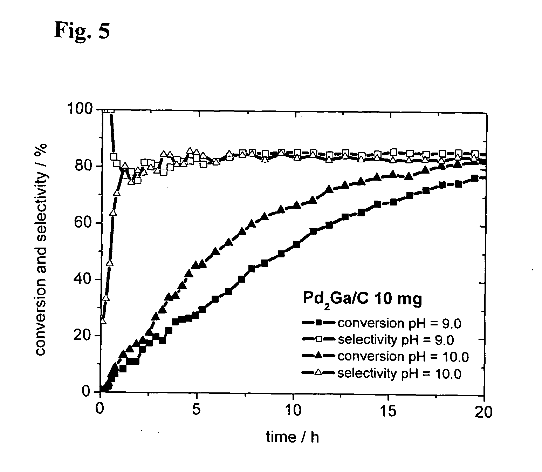

Using an experimental setup as illustrated in Fig. 5, 232 mg $GaI_3$ and 40 mg Ga (corresponding to a Ga : I molar ratio of about 1 : 1.42) were heated in the first reaction zone at a temperature of 873 K. 2 g of a 5 wt.-% Pd/C catalyst obtained from Sigma Aldrich (Catalogue No.: 205680) were heated at a temperature of $T_2$ = 1073 K in the second reaction zone. Through the heterogeneous gas-solid-method a $Pd_2Ga/C$ sample was obtained.

The sample was subsequently etched as follows. 40 mg of the $Pd_2Ga/C$ sample and 40 ml of ammonia solution were used. The pH of the ammonia solution was adjusted with a pH-meter except of the case where a concentrated solution (25% $NH_3$) was used without dilution. Solutions having a pH of 9.0 and 10.0 were used. Portions of the sample were stirred in the respective solution (pH 9 or 10) for 10-15 minutes. Afterwards it was centrifuged (10 minutes, 6000 rpm) in order to separate the solid particles from the liquid solution. The solid catalyst was additionally dried in a desiccator for 2 hours at a pressure of 10 mbar.

Fig. 6 shows the results of the catalytic testing of 10 mg of the obtained carbon-supported $Pd_2Ga$ ($Pd_2Ga/C$) (etched at pH 9.0 and 10.0) in the selective hydrogenation of acetylene as detailed hereinafter.

EXAMPLE 6: Synthesis of PdGa with the heterogeneous gas-solid-method

As illustrated in Fig. 5, 150 mg $GaI_3$ and 76 mg Ga were kept in a first reaction zone at a temperature of 873 K to yield GaI. That is, the Ga : I molar ratio was about 1.43 : 1. 100 mg palladium powder were heated at a temperature of $T_2$ = 1073 K in the second reaction zone. This way, PdGa could be obtained in the second reaction zone in good yield.

COMPARATIVE EXAMPLE 1: Conventional Synthesis of PdGa

1.2083 g palladium (ChemPur 99.95%) and 0.7917 g gallium (ChemPur 99.99%) were molten in glassy carbon crucibles under argon atmosphere in a high-frequency induction furnace to obtain 2 g PdGa (11.354 mmol). After cooling, the solidified molten mass was taken out and subjected to grinding in a mortar.

The crystal structure of the product was controlled by X-ray diffractometry using a STOE STADI P diffractometer (Cu $K_{\alpha 1}$ radiation, curved Ge monochromator) in transmission geometry with a linear position sensitive detector and comparison with reference data from the literature.

Fig. 1 shows the results of the catalytic testing of 400 mg of the obtained PdGa in the selective hydrogenation of acetylene as detailed hereinafter.

COMPARATIVE EXAMPLE 2 Conventional Synthesis of $Pd_2Ga$

$Pd_2Ga$ was obtained by melting the 7.53248 g palladium (ChemPur 99.95 %) and 2.46752 g gallium (ChemPur 99.99 %) while adhering to the experimental protocol provided in Comparative Example 1, above. The obtained material was tempered at 800 °C in an evacuated quartz vial for one week. After taking out and cooling, the solidified molten mass was taken out and subjected to grinding in a mortar.

Again, X-ray diffractometry was used to confirm that the obtained product was $Pd_2Ga$.

Fig. 3 shows the results of the catalytic testing of 10 mg of the obtained $Pd_2Ga$ in the selective hydrogenation of acetylene as detailed hereinafter.

REFERENCE EXAMPLE 1: Preparation of nano-$Cu_{85}Pd_{15}$

0.2384 g (0.9108 mmol) $Cu(acac)_2$ (Acros, 99 %) and 0.0490 g (0.161 mmol) $Pd(acac)_2$ (Fluka, purum) were charged in a three-neck reaction flask, and 15 ml THF were added. A grey-blue suspension was obtained upon switching on the magnetic stirrer. The suspension was heated to the reaction temperature of 50 °C. Subsequently, 2.14 ml (2.14 mmol) Superhydride® solution (1.0 M in THF, Aldrich) were injected in the suspension at a rate of 149.1 ml/h using a perfusor. The suspension immediately turned black, and an evolution of gases was observed. After the Superhydride® addition was complete, the heater and stirrer were switched off. The obtained suspension was subjected to centrifugation at 6000 rpm for 10 minutes. The clear transparent supernatant was decanted, and the black precipitate was washed three times with 2 ml of THF, and finally dried in vacuum at a pressure of 10 mbar. 0.075 g nanoparticulate $Cu_{85}Pd_{15}$ was obtained.

REFERENCE EXAMPLE 2: Preparation of nano-$Cu_{60}Pd_{40}$

For the preparation of the above-captioned compound, the procedure of Reference Example 1 was repeated except that 0.1460 g (0.558 mmol) $Cu(acac)_2$, 0.1132 g (0.372 mmol) $Pd(acac)_2$ and 1.86 ml (0.186 mmol) Superhydride® solution in 15 ml THF were used. 0.075 g nanoparticulate $Cu_{60}Pd_{40}$ was obtained.

CATALYTIC TESTS

Catalytic investigations were performed in a plug flow reactor consisting of a quartz tube with a length of 300 mm, an inside diameter of 7 mm and equipped with a sintered glass frit to support the catalyst bed. For temperature control, a thermocouple was located next to the heating wire wound around the reactor. A second thermocouple was placed inside the reactor to measure the temperature of the catalyst bed. The reactant gases were mixed with Bronkhorst mass flow controllers (total flow 30 ml/min). A Varian CP 4900 Micro gas chromatograph (GC) was used for effluent gas analysis. The Varian MicroGC contains three modules, each with an individual column and a thermal conductivity detector. Hydrogen and helium of the feed gas, and possible oxygen and nitrogen impurities because of leaks in the set-up were separated on a molsieve column. Acetylene, ethylene, and ethane were separated on an alumina column. The total concentration of $C_4$ hydrocarbons (1-butyne, 1-butene, 1,3-butadiene, n-butane, trans and cis-2-butene) was determined using a siloxane (dimethylpolysiloxane) column. Higher hydrocarbons were also separated on the siloxan column but not further quantified because of the presence of many different $C_6$ and $C_8$ hydrocarbons and their low total concentration (less than 0.1% of absolute product stream concentration). Argon (6.0) and helium (6.0) were used as carrier gases for the molsieve column and for the other columns, respectively. A measurement cycle including stabilization, sampling, injection, and separation took between 4 and 5 minutes.

Acetylene hydrogenation experiments were carried out under the condition of 0.5% acetylene, 5% hydrogen, and 50% ethylene in helium. All gases were obtained from Westfalen Gas (Germany).

Activity and selectivity of the materials in the hydrogenation of acetylene were measured by temperature-programmed and by isothermal experiments. The experiments were performed at 473 K in the isothermal mode. The conversion rate was calculated using the following equation:

$$Conv = \frac{(C_{bypass} - C_x)}{C_{bypass}}$$

where $C_x$ is the acetylene concentration in the product stream and $C_{bypass}$ is the acetylene concentration in the feed before the reaction. The selectivity was calculated from the following equation, with $C_{bypass}$ being the acetylene concentration before the reactor and $C_x$ the acetylene concentration after the reactor:

$$Sel = \frac{(C_{bypass} - C_x)}{C_{bypass} - C_x + C_{ethane} + 2xC_{C4Hx}}$$

Calculation of the selectivity assumes that acetylene is only hydrogenated to ethylene, which may be further hydrogenated to ethane. The amount of $C_6$ and higher hydrocarbons, and of carbon deposits formed was supposed to be negligible. In addition to hydrogenation of acetylene to ethane, ethylene from feed may be hydrogenated to ethane, which is included in the selectivity equation. In order to measure selectivity in acetylene hydrogenation at the same conversion, different amounts of catalysts were used according to their specific activity determined in a previous experiment.

Activity of the samples was calculated using the following equation:

$$Act = \frac{Conv\ C_{feed}\ C_{exp}}{m_{cat}}$$

where *Conv* is the calculated acetylene conversion, $C_{feed}$ is the concentration of acetylene in feed, i.e. 0.5 %, $m_{cat}$ the amount of used catalyst in g and constant $C_{exp}$ is 1.904 g/h and contains experimental parameters like total gas flow (30 ml/min), temperature (300 K) and pressure (1013 mbar) and is based on the perfect gas model.

The samples were diluted with 150 mg boron nitride (hexagonal, 99.5%, 325 mesh, Aldrich) prior to conducting the catalytic tests.

RESULTS

Results of the catalytic testing in the selective hydrogenation of acetylene as described above are summarized in Table 1, below.

TABLE 1

| Sample | Sample mass [mg] | Acetylene conversion [%] | Selectivity [%] | Activity [gC$_2$H$_2$/g$_{Pd}$·h] |
|---|---|---|---|---|
| nano-PdGa, Example 1 | 2.5 | 78 | 77 | 4.92 |
| nano-Pd$_2$Ga, Example 2 | 0.1 | 87 | 60 | 110.05 |
| nano-Pd$_2$Ga/SiO$_2$, Example 3 | 0.05 | 98 | 72 | 186.59 |
| nano-Pd$_2$Ga/Al$_2$O$_3$, Example 4 | 0.05 | 97 | 57 | 184.69 |

(continued)

| Sample | Sample mass [mg] | Acetylene conversion [%] | Selectivity [%] | Activity [$gC_2H_2/g_{Pd}\cdot h$] |
|---|---|---|---|---|
| $Pd_2Ga/C$, etched at pH 10, Example 5 | 10 | 82 | 83 | 15.42 |
| PdGa, Comparative Example 1 | 400 | 64 | 77 | 0.025 |
| $Pd_2Ga$, Comparative Example 2 | 10 | 94 | 75 | 1.18 |

Summarizing the results reported in the above table and shown in the figures, nanoparticulate intermetallic Pd/Ga compounds like PdGa and $Pd_2Ga$ prepared by way of the reduction method of the invention were shown to have an activity increase by a factor of about 100 to 200 in the semihydrogenation of acetylene in the presence of an excess of ethylene, in comparison to compounds obtained by melting together the metals. As shown for nanoparticulate $Pd_2Ga$, the selectivity and/or the stability of the catalysts in the semihydrogenation of acetylene can be further increased by suspending the material in an inert liquid in the presence of a powder of an inert material such as silica or alumina, with subsequent drying. This is illustrated in Fig. 4a/b. Moreover, carbon-supported intermetallic Pd/Ga compounds prepared via the heterogeneous gas-solid-method using commercially available carbon-supported palladium as a starting material were shown to be highly active and selective in the semihydrogenation of acetylene.

## Claims

1.   A method of preparing an ordered intermetallic palladium gallium compound comprising the step (1) of reacting a palladium compound and a gallium compound in the presence of a reducing agent.

2.   The method according to Claim 1, wherein step (1) is carried out in a solvent.

3.   The method according to Claim 2, wherein the solvent is tetrahydrofuran or diglyme

4.   The method according to any one of Claims 1 to 3 further comprising the step (2) of isolating the ordered intermetallic palladium gallium compound in powder form.

5.   The method according to any one of Claims 1 to 4, which further comprises a step (3) of tempering the ordered intermetallic palladium gallium compound, optionally following the isolating step (2).

6.   The method according to Claim 5, wherein the tempering in step (3) is carried out at a temperature of 160 to 200 °C, preferably 185 °C.

7.   The method according to Claim 5 or 6, wherein the tempering in step (3) is carried out in a solvent.

8.   The method according to any one of Claims 1 to 7, wherein the palladium compound is a salt of Pd(II), preferably $Pd(acac)_2$.

9.   The method according to any one of Claims 1 to 8, wherein the gallium compound is a gallium halide.

10.   The method according to any one of Claims 1 to 9, wherein the reducing agent is represented by the following formula:

$$M[AR_n(OR')_{3-n}H]_u$$

wherein M represents an alkali metal or alkaline earth metal; A represents Al, Ga or B; R and R' is independently selected from $C_{1-6}$ alkyl, $C_{6-10}$ aryl or $C_{7-16}$ aralkyl; u is 1 when M is an alkali metal and u is 2 when M is an alkaline earth metal, and n is an integer of 0 to 3.

11.   The method according to Claim 10, wherein the reducing agent is represented by the following formula:

$$M(BR_3H)_u$$

wherein M, R and u are as defined in Claim 10.

12. The method according to Claim 10 or 11, wherein the reducing agent is $LiBEt_3H$.

13. The method according to any one of Claims 1 to 12, wherein the ordered intermetallic palladium gallium compound is selected from the group consisting of $PdGa$, $Pd_2Ga$, $PdGa_5$, $Pd_3Ga_7$, $Pd_5Ga_3$, or a mixture thereof.

14. The method according to any one of Claims 4 to 13, wherein the obtained ordered intermetallic palladium gallium compound in powder form is further suspended in an inert liquid in the presence of an inert material and the liquid is subsequently removed.

15. The method according to Claim 14, wherein the inert material is silica or alumina.

16. A method of preparing an ordered intermetallic palladium gallium compound comprising the step of reacting palladium with a gallium compound, wherein the gallium compound is in the vapour phase.

17. The method of Claim 16, wherein the gaseous gallium compound is formed in a first reaction zone at a temperature $T_1$, and the reaction of palladium with the said gaseous gallium compound takes place in a second reaction zone at a temperature $T_2$, wherein $T_2 > T_1$.

18. The method of Claim 16 or 17, wherein the gallium compound is a gallium iodide.

19. The method of any one of Claims 16 to 18, wherein the ordered intermetallic palladium gallium compound is $PdGa$ or $Pd_2Ga$.

20. The method according to any one of Claims 16 to 19, wherein the ordered intermetallic palladium gallium compound is subjected to a subsequent etching treatment, preferably using an alkaline etching solution.

21. The method according to any one of Claims 16 to 20, wherein the palladium is present in powder form.

22. The method according to any one of Claims 16 to 20, which is a method for preparing a supported ordered intermetallic palladium gallium compound, wherein the palladium is used in a form supported on an inert support material.

23. The method according to Claim 22, wherein the inert support material is carbon.

24. A method of preparing a supported ordered intermetallic palladium gallium compound comprising the step of impregnating a support material with a solution or suspension of a palladium compound and a gallium compound, and carrying out a reduction step as defined in Claim 1.

25. An ordered intermetallic palladium gallium compound obtainable by a method according to any one of Claims 1 to 21.

26. The ordered intermetallic palladium gallium compound according to Claim 25 which comprises nanoparticles of the ordered intermetallic compound.

27. The ordered intermetallic palladium gallium compound according to Claim 25 or 26 in powder form.

28. A supported ordered intermetallic palladium gallium compound obtainable by a method according to any one of Claims 22 to 24.

29. The supported ordered intermetallic palladium gallium compound according to Claim 28 which comprises nanoparticles of the ordered intermetallic compound.

30. A use of the ordered intermetallic palladium gallium compound according to any of Claims 25 to 27 as a catalyst.

31. A use of the supported ordered intermetallic palladium gallium compound according to Claim 28 or 29 as a catalyst.

**32.** A process for the selective hydrogenation of an alkyne to give the corresponding alkene, which process comprises reacting a reaction mixture comprising the alkyne with hydrogen in the presence of a hydrogenation catalyst, wherein the hydrogenation catalyst comprises an ordered intermetallic palladium gallium compound according to any one of Claims 25 to 27.

**33.** A process for the selective hydrogenation of an alkyne to give the corresponding alkene, which process comprises reacting a reaction mixture comprising the alkyne with hydrogen in the presence of a hydrogenation catalyst, wherein the hydrogenation catalyst is a supported ordered intermetallic palladium gallium compound according to Claims 28 or 29.

**34.** The process according to Claim 32 or 33, wherein the alkyne is ethyne which is converted to ethene through the selective hydrogenation.

**35.** The process according to Claim 34, wherein the ethyne is present in admixture with an excess of ethene, in the reaction mixture.

**Fig. 1**

**Fig. 2**

**Fig. 3**

**Fig. 4 a**

**Fig. 4 b**

**Fig. 5**

**European Patent Office**

**EUROPEAN SEARCH REPORT**

Application Number

EP 07 02 1904

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KOMATSU T ET AL: "Nano-size particles of palladium intermetallic compounds as catalysts for oxidative acetoxylation" APPLIED CATALYSIS A: GENERAL, ELSEVIER SCIENCE, AMSTERDAM, NL, vol. 251, no. 2, 30 September 2003 (2003-09-30), pages 315-326, XP004458139 ISSN: 0926-860X * abstract * * paragraphs [001.], [02.1] * * page 5, paragraph 3.1; figure 2 * ----- | 1,4,5,8, 24-31 | INV. B01J23/44 B01J23/62 B01J37/02 B01J37/16 C07C5/09 C07C7/167 C22C1/00 |
| X | US 2005/048658 A1 (JOHNSON MARVIN M [US] ET AL) 3 March 2005 (2005-03-03) * claim 1; examples 9,11; tables 1,2 * ----- | 1,4,5,8, 24-35 | |
| X | US 5 364 998 A (SARRAZIN PATRICK [FR] ET AL) 15 November 1994 (1994-11-15) * examples 2,3 * ----- | 1,4,5,8, 24-31 | |
| D,X | M. ARMBRÜSTER, K. KOVNIR, J. OSSWALD, R. GIEDIGKEIT, T. RESSLER , YU. GRIN, R. SCHLÖGL: "PdGa - ein selektiver Katalysator für die Semihydrierung von Acetylen" ZEITSCHRIFT FÜR ANORGANISCHE UND ALLGEMEINE CHEMIE, vol. 632, no. 12-13, 23 August 2006 (2006-08-23), pages 2083-2083, XP002476877 * the whole document * ----- -/-- | 25,27, 30,32, 34,35 | TECHNICAL FIELDS SEARCHED (IPC) B01J C07C C22C |

The present search report has been drawn up for all claims

| Place of search | Date of completion of the search | Examiner |
|---|---|---|
| Munich | 17 June 2008 | Holzwarth, Arnold |

EPO FORM 1503 03.82 (P04C01)

**European Patent Office**

## EUROPEAN SEARCH REPORT

Application Number

EP 07 02 1904

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | KOVNIR K ET AL: "PdGa and Pd3Ga7: Highly-Selective catalysts for the acetylene partial hydrogenation" STUDIES IN SURFACE SCIENCE AND CATALYSIS, ELSEVIER SCIENCE B.V., AMSTERDAM, NL, vol. 162, 2006, pages 481-488, XP009097585 ISSN: 0167-2991 * abstract * * paragraphs [003.], [004.]; figure 1 * ----- | 25,27, 30,32, 34,35 | |
| D,X | EP 1 834 939 A (MPG MAX PLANCK GES ZUR FOERDER [DE]) 19 September 2007 (2007-09-19) * examples 1-4; table 1 * ----- | 25,27, 30,32, 34,35 | |
| X | WANNEK C ET AL: "Phase equilibria in the palladium-rich part of the gallium-palladium system. The crystal structures of Ga3Pd7 and Ga1-xPd2+x" JOURNAL OF ALLOYS AND COMPOUNDS, ELSEVIER SEQUOIA, LAUSANNE, CH, vol. 316, no. 1-2, 2 March 2001 (2001-03-02), pages 99-106, XP004317507 ISSN: 0925-8388 * abstract * * paragraph [02.1] * ----- -/-- | 16,18, 19,21, 25,27, 30,32, 34,35 | TECHNICAL FIELDS SEARCHED (IPC) |

The present search report has been drawn up for all claims

| Place of search | Date of completion of the search | Examiner |
|---|---|---|
| Munich | 17 June 2008 | Holzwarth, Arnold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**European Patent Office**

## EUROPEAN SEARCH REPORT

Application Number

EP 07 02 1904

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CH. WANNEK, B. HARBRECHT: "Die Kristallstruktur von Ga5Pd13 - eine niedersymmetrische Ordnungsvariante der kubisch dichtesten Kugelpackung" ZEITSCHRIFT FÜR ANORGANISCHE UND ALLGEMEINE CHEMIE, vol. 626, no. 7, 7 July 2000 (2000-07-07), pages 1540-1544, XP002476878 * abstract * * page 1540, paragraph SYNTHESE * * page 1543, paragraph EXPERIMENTELLES * | 16,18, 19,21, 25,27, 30,32, 34,35 | |
| A | R. NEDDERMANN, R. WARTCHOW, M. BINNEWIES: "Chemischer Transport intermetallischer Phasen. 6 [1] Der Chemische Transport von Cr(Ge) (Mischkristall), Cr3Ge, Cr5Ge3, Cr11Ge8, CrGe, Cr11Ge19 und Ge(Cr) (Mischkristall)" ZEITSCHRIFT FÜR ANORGANISCHE UND ALLGEMEINE CHEMIE, vol. 624, no. 4, 25 February 1999 (1999-02-25), pages 733-736, XP002477339 * paragraph [0002] * | 16,18, 19,21, 25,27, 30,32, 34,35 | TECHNICAL FIELDS SEARCHED (IPC) |
| A | BINNEWEIS M: "CHEMISCHE TRANSPORTREAKTIONEN" CHEMIE IN UNSERER ZEIT, VERLAG CHEMIE, WEINHEIM, DE, vol. 32, no. 1, 1998, pages 15-21, XP009083674 ISSN: 0009-2851 * figure 1 * | 16,18, 19,21, 25,27, 30,32, 34,35 | |

The present search report has been drawn up for all claims

| Place of search | Date of completion of the search | Examiner |
|---|---|---|
| Munich | 17 June 2008 | Holzwarth, Arnold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

Application Number

EP 07 02 1904

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

| | | |
|---|---|---|
| **European Patent Office** | **LACK OF UNITY OF INVENTION SHEET B** | Application Number<br>EP 07 02 1904 |

The Search Division considers that the present European patent application does not comply with the requirements of unity of invention and relates to several inventions or groups of inventions, namely:

1. claims: 1-15,24,25 (part.)-35(part.)

                    Claims 1-15, 24:
        A method preparing an ordered intermetallic palladium
        gallium compound by reacting a palladium compound and a
        gallium compound in the presence of a reducing agent.
        Claims 25 (part.)-29 (part.):
        An ordered intermetallic palladium gallium compound
        obtainable by said method.
        Claims 30 (part.)-31 (part.):
        A use of said ordered intermetallic palladium gallium
        compound as a catalyst.
        Claims 32 (part.)-35 (part.):
        A process for the selective hydrogenation of an alkyne to
        give the corresponding alkene using said ordered
        intermetallic palladium gallium compound as a catalyst
        .

                        ---

2. claims: 16-23,25 (part.)-35(part.)

                    Claims 16-23:
        A method preparing an ordered intermetallic palladium
        gallium compound by reacting palladium and a gallium
        compound, wherein the gallium compound is in the vapour
        phase.
        Claims 25 (part.)-29 (part.):
        An ordered intermetallic palladium gallium compound
        obtainable by said method.
        Claims 30 (part.)-31 (part.):
        A use of said ordered intermetallic palladium gallium
        compound as a catalyst.
        Claims 32 (part.)-35 (part.):
        A process for the selective hydrogenation of an alkyne to
        give the corresponding alkene using said ordered
        intermetallic palladium gallium compound as a catalyst
                        ---

**ANNEX TO THE EUROPEAN SEARCH REPORT
ON EUROPEAN PATENT APPLICATION NO.**

EP 07 02 1904

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2008

| Patent document cited in search report | | Publication date | Patent family member(s) | | Publication date |
|---|---|---|---|---|---|
| US 2005048658 | A1 | 03-03-2005 | AU | 2003298040 A1 | 06-04-2005 |
| | | | EP | 1677910 A1 | 12-07-2006 |
| | | | WO | 2005025746 A1 | 24-03-2005 |
| US 5364998 | A | 15-11-1994 | CA | 2093273 A1 | 03-10-1993 |
| | | | CN | 1081664 A | 09-02-1994 |
| | | | DE | 69311192 D1 | 10-07-1997 |
| | | | DE | 69311192 T2 | 08-01-1998 |
| | | | EP | 0564328 A1 | 06-10-1993 |
| | | | ES | 2105150 T3 | 16-10-1997 |
| | | | FR | 2689517 A1 | 08-10-1993 |
| | | | IN | 181363 A1 | 30-05-1998 |
| | | | JP | 6025020 A | 01-02-1994 |
| EP 1834939 | A | 19-09-2007 | WO | 2007104569 A1 | 20-09-2007 |

EPO FORM P0459

For more details about this annex : see Official Journal of the European Patent Office, No. 12/82

## REFERENCES CITED IN THE DESCRIPTION

### Patent documents cited in the description

- EP 1834939 A
- WO 2007104569 A
- DE 3943351 A
- EP 0423627 A
- WO 03106020 A

### Non-patent literature cited in the description

- **E. HELLNER et al.** *Z. Naturforsch.,* 1947, vol. 2a, 177-183
- **K. KHALAFF et al.** *J. Less-Common Met.,* 1974, vol. 37, 129-140
- **K. KOVNIR et al.** *Stud. Surf. Sci. Catal.,* 2006, vol. 162, 481-488
- **M. ARMBRÜSTER et al.** *Z. Anorg. Allg. Chem.,* 2006, vol. 632, 2083
- **H. BÖNNEMANN et al.** *Angew. Chem. Int. Ed. Engl.,* 1990, vol. 29, 273-275
- *J. Mol. Catal.,* 1994, vol. 86, 129-177
- **H. BÖNNEMANN.** *Langmuir,* 1998, vol. 14, 6654-6657
- **K. PAGE.** *Materials Research Bulletin,* 2007, vol. 42, 1969-1975
- **J. S. BRADLEY.** *Chem. Mater.,* 1993, vol. 5, 254-256
- **S. ILLY-CHERREY et al.** *Materials Science and Engineering,* 2000, vol. A283, 11-16
- **H. SCHÄFER et al.** *Z. anorg. allg. Chem.,* 1975, vol. 414, 137-150
- **M. BINNEWIES.** *Angew. Chem.,* 2001, vol. 113, 3801-3803
- **P. BINGER et al.** *Liebigs Ann. Chem.,* 1968, vol. 717, 21-40